# EUROPEAN PATENT APPLICATION

(11) **EP 4 371 577 A1**
(43) Date of publication of application: **22.05.2024**
(21) Application number: 22842379.4
(22) Date of filing: 08.07.2022
(51) Int. Cl.: A61K 47/54, A61K 9/00, A61K 47/44, A61K 47/14, A61K 47/69, A61K 38/22, A61K 38/26, A61P 3/10

(54) **COMPOSITION FOR ORALLY ADMINISTERED FORMULATION CONTAINING GLP-1 ANALOGUE**

(30) Priority: 12.07.2021 KR 20210091200
(71) Applicant: Hanmi Pharm. Co., Ltd., Hwaseong-si, Gyeonggi-do 18536 (KR)
(72) Inventor: CHOI, Go-Wun, Hwaseong-si, Gyeonggi-do 18536 (KR); CHO, Hyuk-Jun, Hwaseong-si, Gyeonggi-do 18536 (KR); IM, Ho-Taek, Hwaseong-si, Gyeonggi-do 18536 (KR); KIM, Yong-Il, Hwaseong-si, Gyeonggi-do 18536 (KR)
(74) Representative: J A Kemp LLP
(86) International application number: PCT/KR2022/009915
(87) International publication number: WO 2023/287117

(57) **Abstract**

The present disclosure relates to a composition, for an orally administered formulation, containing a GLP-1 analogue and, particularly, to a pharmaceutical composition for oral administration comprising a hydrophobic ion-pair of a GLP-1 analogue and an oil phase, and a method for preparing the composition. The pharmaceutical composition for oral administration of the present disclosure forms an emulsion surrounding the hydrophobic ion-pair when exposed to a water phase, so as to be stably absorbed without being decomposed by means of digestive enzymes, and thus provides a pharmacological effect. Therefore, the present disclosure may be utilized as a pharmaceutical composition for oral administration.

## Description

### [Technical Field]

The present disclosure relates to a composition for an orally administered formulation, containing a GLP-1 analogue, and particularly, to a pharmaceutical composition for oral administration comprising a hydrophobic ion-pair of a GLP-1 analogue and an oil phase, and a method for preparing the composition.

### [Background Art]

Glucagon-like peptide-1 (GLP-1) is a hormone composed of 30 amino acids and is known to have therapeutic effects on diabetes by promoting insulin secretion from beta cells of the pancreas and suppressing glucagon secretion from alpha cells, and the like. However, GLP-1 and GLP-1 analogues developed to improve activity and stability thereof have problems in that they are mainly administered by parenteral routes such as intravenous or subcutaneous injection, resulting in significantly reduced dosing convenience and occurrence of rash and swelling at the injection site.

The GLP-1 analogues, which are peptide drugs, have problems of being decomposed by acids and digestive enzymes in the gastrointestinal tract when administered orally, and also having poor absorption in the body. However, so far, no formulation for oral administration has been developed that is stable in the body after administration, while having excellent bioavailability. As an oral formulation of GLP-1 or a GLP-1 analogue, Korean Patent Laid-Open Publication No. 2020-0076642 discloses a preparation containing polyoxylglyceride, acylglycerol complex, and polyethylene glycol as excipients, Korean Patent Laid-Open Publication No. 2020-0081411 discloses a preparation containing sodium chenodeoxycholate and/or propyl gallate as excipients, and Korean Patent Laid-Open Publication No. 2016-0002945 discloses a preparation containing a salt of N-(8-2-hydroxybenzoyl)amino)caprylic acid (SNAC). However, a preparation for oral administration that is competitive compared to existing injection preparations has not yet been developed. Novo Nordisk's 'Rybelsus^{®}' approved by the U.S. FDA and is currently on the market, was expected to replace the existing GLP-1 injections, however, 'Ribelsus^{®}' has disadvantages of lower efficacy compared to existing injections, the need to be taken daily 30 minutes before taking water, food, etc., and inconvenient dosing that splitting or chewing the medicine is not allowed. Thus, there is a continuous need to develop another preparation for oral administration.

Under this background, the present inventors have made diligent efforts to develop a composition for oral administration containing a GLP-1 analogue, and as a result, developed a composition obtained by binding the GLP-1 analogue and a counter ion through electrostatic attraction to form a hydrophobic ion-pair, and dissolving the hydrophobic ion-pair in an oil phase containing oil or a surfactant, and completed the present disclosure by confirming that the composition could form an emulsion in the gastrointestinal tract when administered orally, thereby preventing the decomposition of the GLP-1 analogue.

### [Related-art Documents]

### [Patent Documents]

(Patent Document 0001) Korean Patent Laid-Open Publication No. 2020-0076642
(Patent Document 0002) Korean Patent Laid-Open Publication No. 2020-0081411
(Patent Document 0003) Korean Patent Laid-Open Publication No. 2016-0002945

### [Disclosure]

### [Technical Problem]

An object of the present disclosure is to provide a pharmaceutical composition for oral administration, comprising: (a) a hydrophobic ion-pair formed by binding a GLP-1 analogue and a counter ion, and (b) an oil phase containing oil or a surfactant.

Another object of the present disclosure is to provide a method for preparing the pharmaceutical composition for oral administration.

### [Technical Solution]

The present disclosure will be described in detail as follows. Meanwhile, each description and embodiment disclosed in the present disclosure may be applied to each of the other descriptions and embodiments. In other words, all combinations of various elements disclosed in the present disclosure fall within the scope of the present disclosure. In addition, it cannot be considered that the scope of the present disclosure is limited by specific descriptions described below.

In an aspect of the present disclosure to achieve the objective, there is provided a pharmaceutical composition for oral administration, comprising: (a) a hydrophobic ion-pair formed by binding a GLP-1 analogue to a counter ion, and (b) an oil phase containing oil or a surfactant.

The pharmaceutical composition for oral administration of the present disclosure may comprise the hydrophobic ion-pair added to the oil phase, the hydrophobic ion-pair being formed by binding the GLP-1 analogue to the counter ion, and may form an emulsion surrounding the hydrophobic ion-pair when the composition is exposed to a water phase, thereby being stably absorbed without being decomposed by digestive enzymes, resulting in providing a pharmacological effect. Therefore, the pharmaceutical composition of the present disclosure may be usefully employed as a pharmaceutical composition for oral administration.

In the present disclosure, "GLP-1 analogues" refer to peptides and variants thereof that bind to the GLP-1 receptor and exhibit the same pharmacological activities as GLP-1. As an example, in the present disclosure, the GLP-1 analogue may be exendin-4 or exendin-4 derivatives, wherein the exendin-4 derivative may include CA-exendin-4 (Imidazoacetyl-exendin-4), DA-exendin-4 (Desamino-histidyl-exendin-4), HY-exendin-4 (beta-hydroxy imidazopropionyl-exendin-4), CX-exendin-4 (beta-carboxyimidazopropionyl-exendin-4), DM-exendin-4 (Dimethyl-histidyl-exendin-4), lixisenatide, liraglutide, semaglutide, dulaglutide, and/or albiglutide, but is not limited thereto.

The exendin-4 is a peptide that exists in the salivary secretion of Heloderma suspectum (America's venomous lizard) and shows a high sequence homology of 53% with the GLP-1 sequence (SEQ ID NO: 1). It has been reported that exendin-4 is able to act on the GLP-1 receptor on insulin-secreting cells, and has higher physiological activity than GLP-1 along with resistance to DPP IV, and the half-life in the body of 2-4 hours, which is longer than that of GLP-1 (US 5, 424, 686). In the present disclosure, exendin-4 may be natural or artificially synthesized.

As an embodiment of the present disclosure, the exendin-4 derivative may be a derivative in which His, the first amino acid in exendin-4, is chemically mutated. All of the details described in Korea Patent No. 10-1231431, which discloses derivatives of exendin-4, are incorporated herein by reference. Specifically, the exendin-4 derivative may include imidazoacetyl-exendin-4 (CA-exendin-4) in which the alpha carbon is removed from the first amino acid, His, desamino-histidyl-exendin-4 (DA-exendin-4) in which the N-terminal amino group is removed therefrom, beta-hydroxy imidazopropionyl-exendin-4 (HY-exendin-4) and beta-carboxy imidazopropyl-exendin-4 (CX-exendin-4) substituted with a hydroxyl or carboxyl group, or dimethyl-histidyl-exendin-4 (DM-exendin-4) modified with two methyl residues, but is not limited thereto. The exendin-4 derivatives have the same amino acid sequence structure as exendin-4, with only some modifications made in the first amino acid, His, and thus all have the same peptide structure and electrical properties. The present disclosure is characterized by forming ion-pairs between the GLP-1 analogue and the counter ion by electrostatic attraction, and thus all peptides and variants thereof having the same peptide structure and electrical properties as the GLP-1 analogue of the present disclosure are also included within the scope of the present disclosure.

As other embodiments of the present disclosure, the GLP-1 analogue may be lixisenatide in which the 38th proline is removed from exendin-4 and 6 lysine residues are added thereto, liraglutide in which the 27th lysine in human GLP-1 is substituted with arginine and a hexadecanoyl group is attached to the remaining lysine through a glutamic acid spacer, semaglutide in which the 8th alanine in GLP-1 is substituted with 2-aminoisobutyric acid, the 34th lysine is substituted with arginine, and the 26th lysine is acylated with stearic diacid, dulaglutide in which GLP-1 (7-37) is linked to the Fc fragment of human IgG4, or albiglutide in which 2 copies of GLP-1 variant, the second alanine in human GLP-1 is replaced with glycine, are composed of amino acid sequences 1-30 and 31-60, and additionally fused to human albumin, but is not limited thereto.

In the present disclosure, 'counter ion' refers to an ion capable of forming a hydrophobic ion-pair in solid form by binding to the GLP-1 analogue by electrostatic attraction. As an example, the counter ion may include anions of 1-hydroxy-2-naphthoic acid (xinafoic acid), 2-naphthalene sulfonic acid (NSA), brilliant blue FCF, carboxy methyl polyethylene glycol (CM-PEG), cholesteryl hemisuccinate, cholic acid, sodium cholate, decanoic acid, sodium decanoate, sodium caprate, dimyristoyl phosphatidylglycerol (DMPG), dioleoyl phosphatidic acid (DOPA), docosahexaenoic acid, hexadecyl phosphate, linoleic acid, N,N-dipalmitoyl-L-lysine, oleic acid, sodium oleate, pamoic acid, disodium pamoate, sodium acetate, sodium cholesteryl sulfate, sodium decanesulfonate (SDES), sodium deoxycholate, sodium docusate, sodium dodecyl benzenesulfonate (SDBS), sodium dodecyl sulfate, sodium laurate, sodium n-octadecyl sulfate, sodium stearate, sodium stearoyl glutamate (SSG), sodium taurodeoxycholate (STDC), sodium tetradecyl sulfate, sodium tripolyphosphate, taurocholic acid, sodium taurocholate, and/or vitamin E succinate.

As another example, the counter ion may include cations of arginine-hexadecanoyl ester (AHE), arginine-nonyl ester (ANE), N-benzyl-2-phenylethanamine, chitosan, dodecylamine, hexadecyl trimethylammonium bromide (CTAB), maprotiline, N^{α}-deoxycholyl-L-lysyl-methylester, N,N'-dibenzyl ethylenediamine, N,N-dimethyl dodecylamine, N,N-dimethyl hexylamine, N,N-dimethyl octadecylamine, stearylamine, tetrabutyl ammonium bromide (TBAB), tetraheptyl ammonium bromide (THA), tetrahexyl ammonium bromide, tetraoctyl ammonium bromide (TOAB), tetrapentyl ammonium bromide (TPA), and/or triethylamine (TEA), but is not limited thereto.

The GLP-1 analogue of the present disclosure is characterized by being added to the oil phase after forming the hydrophobic ion-pair with the counter ion. Specifically, the oil phase containing oil or a surfactant may comprise: (a) polyoxyl castor oil having an average number of oxyethylene units of 38 or less, and (b) at least any one selected from the group consisting of oleoyl macrogolglyceride, propylene glycol fatty acid ester, propylene glycol, diethylene glycol monoethyl ether, mono-di-glyceride, caprylocaproyl polyoxylglyceride, and polyoxyethylene sorbitan fatty acid ester, but is not limited thereto.

In the present disclosure, the oil phase may further comprise an absorption enhancer for improving the absorption of the GLP-1 analogue into the body. The absorption enhancer may be, for example, EDTA, citric acid, salicylate, SDS, sodium deoxycholate, sodium taurocholate, oleic acid, acylcarnitine, sodium caprate, sodium caprylate, salcaprozate (SNAC), 5-CNAC, dimyristoyl phosphatidylglycerol (DMPG), cetyltrimethylammonium bromide (CTAB) and/or phospholipids, and any absorption enhancer that improves the absorption of GLP-1 analogues into the body is included within the scope of the present disclosure.

The pharmaceutical composition may be usefully employed, for example, for preventing or treating diabetes. The therapeutic effect and pharmacological mechanism of GLP-1 analogues such as exendin-4 on diabetes are already well known in the art.

Also, in another aspect of the present disclosure, there is provided a method for treating or preventing diabetes, comprising administering a therapeutically effective amount of the pharmaceutical composition to a subject in need thereof. The subject may be a mammal including a human.

As used in the present disclosure, the term "therapeutically effective amount" refers to the amount of the pharmaceutical composition effective for treating or preventing diabetes. Specifically, the "therapeutically effective amount" means an amount sufficient to treat a disease at a reasonable benefit/risk ratio applicable to medical treatment, and the effective dose level may be determined depending on factors including the subject type and severity, age, sex, type of diseases, the activity of the drug, the sensitivity to the drug, the time of administration, the route of administration, the rate of excretion, the duration of treatment, drugs used concurrently, and other factors well known in the medical field. The pharmaceutical composition of the present disclosure may be administered as an individual therapeutic agent or in combination with other therapeutic agents, or may be administered sequentially or simultaneously with a commercially available therapeutic agent. In addition, the pharmaceutical composition of the present disclosure may be administered singly or in multiple doses. In consideration of all of the above factors, it is important to administer an amount capable of obtaining the maximum effect with the minimum amount without side effects, which may be easily determined by those skilled in the art. The administration dose of the pharmaceutical composition of the present disclosure may be determined by specialists according to various factors such as the patient's condition, age, sex, complications, and the like. Since the active ingredient of the pharmaceutical composition of the present disclosure has excellent safety, the active ingredient may be used even above the predetermined dose.

Also, in another aspect of the present disclosure, the present disclosure provides use of the pharmaceutical composition for the manufacture of a medicament for use in the treatment or prevention of diabetes. The pharmaceutical composition for the manufacture of a medicament may be mixed with acceptable adjuvants, diluents, carriers, and the like, and may be prepared as a combined preparation with other active agents to have a synergistic effect of the active ingredients.

Matters described in the use, composition, and treatment method of the present disclosure are equally applied unless they contradict each other.

In another aspect of the present disclosure to achieve the objective, there is provided a method for preparing a pharmaceutical composition for oral administration, comprising: (a) preparing a hydrophobic ion-pair by mixing a GLP-1 analogue with a counter ion; and (b) mixing the hydrophobic ion-pair into an oil phase.

The GLP-1 analogue, counter ion, hydrophobic ion-pair, and oil phase were the same as described above.

In the present disclosure, the Step (a) is to prepare the GLP-1 analogue as a hydrophobic ion-pair in solid form, and specifically may comprise (a-1) dissolving the GLP-1 analogue and the counter anion in an aqueous solution at pH 4.0, respectively; (a-2) adding the counter anion solution dropwise to the GLP-1 analogue solution; and (a-3) recovering the ion-pair from a mixture of the Step (a-2). Otherwise, the Step (a) may comprise (a-4) dissolving the GLP-1 analogue in an aqueous solution at pH 8.0 and dissolving the counter cation in an aqueous methanol solution; (a-5) adding the counter anion solution dropwise to the GLP-1 analogue solution; and (a-6) recovering the ion-pair from a mixture of the Step (a-5), but is not limited thereto.

Another aspect of the present disclosure is a method for preparing a pharmaceutical composition for oral administration, comprising: (a) preparing a hydrophobic ion-pair by mixing a GLP-1 analogue with a counter ion; (b) adding the hydrophobic ion-pair to a mixture of medium chain triglyceride and propylene glycol; and (c) adding and mixing at least any one selected from the group consisting of polyoxyl 35 castor oil, polyoxyl 40 hydrogenated castor oil, oleoyl macrogolglyceride, and propylene glycol fatty acid ester, to a mixture prepared in the Step (b).

Another aspect of the present disclosure is a method for preparing a pharmaceutical composition for oral administration, comprising: (a) preparing a hydrophobic ion-pair by mixing a GLP-1 analogue with a counter ion;
(b) adding the hydrophobic ion-pair to a mixture of propylene glycol fatty acid ester and propylene glycol; and
(c) adding and mixing at least any one selected from the group consisting of medium chain triglyceride, polyoxyl 35 castor oil, polyoxyl 40 hydrogenated castor oil, and oleoyl macrogolglyceride, to a mixture prepared in the Step (b) .

In preparing the pharmaceutical composition for oral administration of the present disclosure, in some cases, it is possible to increase the stability of gastrointestinal enzymatic degradation by first adding hydrophobic ion-pairs to a mixture of propylene glycol, a hydrophilic co-solvent, and medium chain triglyceride or propylene glycol fatty acid ester to minimize dispersion, followed by mixing remaining oil components, compared to when simply adding hydrophobic ion-pairs to an oil mixture.

Another aspect of the present disclosure is a pharmaceutical composition for oral administration prepared by the preparation method as described above.

Exemplary embodiments of the present disclosure may be modified in various other forms, and the scope of the present disclosure is not limited to the exemplary embodiments described below. In addition, the exemplary embodiments of the present disclosure are provided to more completely explain the present disclosure to an ordinary person skilled in the art. Further, "including" a component throughout the specification does not mean excluding other components, but rather it means that other components may be further included, unless otherwise stated.

### [Advantageous Effects]

When exposed to a water phase, the pharmaceutical composition for oral administration of the present disclosure may form an emulsion surrounding hydrophobic ion-pairs to be stably absorbed without being decomposed by digestive enzymes, thereby exhibiting a pharmacological effect. Therefore, the composition of the present disclosure may be usefully employed as a pharmaceutical composition for oral administration.

### [Description of Drawings]

FIG. 1 shows test results of stability of compositions of Examples 1 to 2, and Comparative Examples 1 to 3 in simulated gastric fluid.
FIG. 2 shows test results of stability of the compositions of Examples 1 to 2, and Comparative Examples 1 to 3 in simulated intestinal fluid.
FIG. 3 shows test results of stability of compositions of Examples 3 and 4, and Comparative Examples 4 to 6 in simulated gastric fluid.
FIG. 4 shows test results of stability of the compositions of Examples 3 and 4, and Comparative Examples 4 to 6 in simulated intestinal fluid.
FIG. 5 shows test results of stability of compositions of Examples 5 to 7 in simulated gastric fluid.
FIG. 6 shows test results of stability of compositions of Comparative Examples 7 to 9 in simulated gastric fluid.
FIG. 7 shows test results of stability of the compositions of Examples 5 to 7 in simulated intestinal fluid.
FIG. 8 shows test results of stability of the compositions of Comparative Examples 7 to 9 in simulated intestinal fluid.
FIG. 9 shows test results of stability of compositions of Comparative Examples 10 and 11 in simulated gastric fluid.
FIG. 10 shows test results of stability of the compositions of Comparative Examples 10 and 11 in simulated intestinal fluid.

### [Best Mode]

Hereinafter, the constitution and effects of the present disclosure will be described in more detail through the following Examples. These Examples are only provided for illustrating the present disclosure, but the scope of the present disclosure is not limited by these Examples.

### Examples 1 to 2: Preparation of CA-exendin-4-containing composition for oral administration

As Examples 1 and 2, compositions for oral administration containing CA-exendin-4, a GLP-1 analogue, were prepared.

To prepare hydrophobic ion-pair solid compositions formed by the electrostatic attraction of CA-exendin-4 and the counter ion, CA-exendin-4 and sodium docusate were each dissolved in a pH 4.0 solution to form cations and anions. The dissolved sodium docusate solution was added dropwise to the CA-exendin-4 solution to obtain hydrophobic solid compositions.

The solid compositions were dissolved in the oil phase (mixture of oil or surfactants) specified in Table 1 to obtain the final pharmaceutical compositions.

**[Table 1]**

| **mg/C** | | Example 1 | Example 2 |
|---|---|---|---|
| **Hydrophobi c ion-pair** | CA-exendin-4 | 0.15 | 0.15 |
| | Sodium docusate | 0.06 | 0.06 |
| | Acetic acid aqueous solution (pH 4.0) | (10) | (10) |
| **Total ion-pairs** | | **0.21** | **0.21** |
| **Oil phase** | Sodium taurocholate | - | 41.0 |
| | Polyoxyl 35 castor oil | 90.0 | 90.0 |
| | Oleoyl macrogolglyceride | 45.0 | 45.0 |
| | Propylene glycol fatty acid ester | 45.0 | 45.0 |
| | Propylene glycol | 25.0 | 25.0 |
| **Total oil phase** | | **205.0** | **246.0** |
| **Sum** | | **205.2** | **246.2** |

| | | | |
|---|---|---|---|
| * Sodium taurocholate is used as an absorption enhancer. | | | |

### Examples 3 to 7: Preparation of exendin-4-containing composition for oral administration

As Examples 3 and 4, compositions for oral administration containing exendin-4, a GLP-1 analogue, were prepared.

To prepare hydrophobic ion-pair solid compositions formed by the electrostatic attraction of exendin-4 and the counter ion, exendin-4 and tetraheptylammonium bromide were each dissolved in a pH 8.0 solution and 50% methanol solution to form cations and anions. The dissolved tetraheptylammonium bromide solution was added dropwise to the exendin-4 solution to obtain hydrophobic solid compositions.

The solid compositions were dissolved in the oil phase (mixture of oil or surfactants) specified in Table 2 to obtain the final pharmaceutical compositions.

**[Table 2]**

| mg/C | | Example 3 | Example 4 |
|---|---|---|---|
| **Hydrophobic ion-pair** | exendin-4 | 1.1 | 1.1 |
| | Tetraheptylammonium bromide | 0.9 | 0.9 |
| | Aqueous sodium hydroxide solution (pH 8.0) | (5) | (5) |
| | 50% methanol | (5) | (5) |
| **Total ion-pairs** | | **2.0** | **2.0** |
| **Oil phase** | Sodium taurocholate | - | 41.0 |
| | Diethylene glycol monoethyl ether | 20.0 | 20.0 |
| | Polyoxyl 35 castor oil | 50.0 | 50.0 |
| | Mono-di-glyceride | 40.0 | 40.0 |
| | Caprylocaproyl polyoxylglyceride | 35.0 | 35.0 |
| | Polyoxyethylene sorbitan fatty acid ester | 35.0 | 35.0 |
| **Total oil phase** | | **180.0** | **221.0** |
| **Sum** | | **182.0** | **223.0** |

| | | | |
|---|---|---|---|
| * Sodium taurocholate is used as an absorption enhancer. | | | |

As Examples 5 to 7, compositions for oral administration containing exendin-4, a GLP-1 analogue, were prepared.

To prepare hydrophobic ion-pair solid compositions formed by the electrostatic attraction of exendin-4 and the counter ion, exendin-4 and tetraheptylammonium bromide were each dissolved in a pH 8.0 solution and 50% methanol solution to form cations and anions. The dissolved tetraheptylammonium bromide solution was added dropwise to the exendin-4 solution to obtain hydrophobic solid compositions.

The solid compositions were first added to a mixture of medium chain triglyceride (MCT) or propylene glycol fatty acid ester and propylene glycol, a hydrophilic co-solvent, and then the remaining oil specified in Table 3 was added to obtain the final pharmaceutical compositions.

**[Table 3]**

| **mg/C** | | Example 5 | Example 6 | Example 7 |
|---|---|---|---|---|
| **Hydrophobic ion-pair** | exendin-4 | 1.03 | 1.03 | 1.03 |
| | Tetraheptylammonium bromide | 0.97 | 0.97 | 0.97 |
| **Total ion-pairs** | | **2.00** | **2.00** | **2.00** |
| **Oil phase** | Medium chain triglyceride | 15.0 | 60.0 | - |
| | Polyoxyl 40 hydrogenated castor oil | 40.0 | 160.0 | 160.0 |
| | Oleoyl macrogolglyceride | - | - | 60.0 |
| | Propylene glycol fatty acid ester | 41.0 | 164.0 | 164.0 |
| **Hydrophilic co-solvent** | Propylene glycol | 4.0 | 16.0 | 16.0 |
| **Total oil phase** | | **100.0** | **400.0** | **400.0** |
| **Sum** | | **102.0** | **402.0** | **402.0** |

### Comparative Example 1

As Comparative Example 1, an aqueous solution of CA-exendin-4 (30 µg/mL) was used.

### Comparative Example 2: Preparation of CA-exendin-4-containing composition without forming ion-pairs

As Comparative Example 2, a composition in which CA-exendin-4 was directly mixed into the oil phase without performing the process for forming hydrophobic ion-pairs was used.

**[Table 4]**

| **mg/c** | | Comparative Example 2 |
|---|---|---|
| **Oil phase** | CA-exendin-4 | **0.15** |
| | Polyoxyl 35 castor oil | 90.0 |
| | Oleoyl macrogolglyceride | 45.0 |
| | Polyethylene glycol fatty acid ester | 45.0 |
| | Propylene glycol | 25.0 |
| **Total oil phase** | | **205.15** |
| **Sum** | | **205.15** |

### Comparative Example 3: Preparation of CA-exendin-4 ion-pair-containing composition without oil phase

As Comparative Example 3, a CA-exendin-4 ion-pair solid composition prepared by electrostatic attraction of hydrophobic ion-pairs was prepared.

**[Table 5]**

| **mg/c** | | Comparative Example 3 |
|---|---|---|
| **Ion-pairs** | CA-exendin-4 | 0.15 |
| | Sodium docusate | 0.06 |
| | Acetic acid aqueous solution (pH 4.0) | (10) |
| **Total ion-pairs** | | **0.21** |
| **Sum** | | **0.21** |

### Comparative Example 4

As Comparative Example 4, an aqueous solution of exendin-4 (30 µg/mL) was used.

### Comparative Example 5: Preparation of exendin-4-containing composition without forming ion-pairs

As Comparative Example 5, a composition in which exendin-4 was directly mixed into the oil phase without performing the process for forming hydrophobic ion-pairs was used.

**[Table 6]**

| **mg/C** | | Comparative Example 5 |
|---|---|---|
| **Oil phase** | exendin-4 | 1.1 |
| | Diethylene glycol monoethyl ether | 20.0 |
| | Polyoxyl 35 castor oil | 50.0 |
| | Mono-di-glyceride | 40.0 |
| | Caprylocaproyl polyoxylglyceride | 35.0 |
| | Polyoxyethylene sorbitan fatty acid ester | 35.0 |
| **Total oil phase** | | **181.1** |
| **Sum** | | **181.1** |

### Comparative Example 6: Preparation of exendin-4 ion-pair-containing composition without oil phase

As Comparative Example 6, an exendin-4 ion-pair solid composition prepared by electrostatic attraction of hydrophobic ion-pairs was prepared.

**[Table 7]**

| **mg/C** | | Comparative Example 6 |
|---|---|---|
| **Ion-pairs** | exendin-4 | 1.1 |
| | Tetraheptylammonium bromide | 0.9 |
| | Aqueous sodium hydroxide solution (pH 8.0) | (5) |
| | 50% methanol | (5) |
| **Total ion-pairs** | | **2.0** |
| **Sum** | | **2.0** |

### Comparative Examples 7 to 11: Preparation of exendin-4-containing composition for oral administration

As Comparative Examples 7 to 11, compositions for oral administration containing exendin-4, a GLP-1 analogue, were prepared.

To prepare hydrophobic ion-pair solid compositions formed by the electrostatic attraction of exendin-4 and the counter ion, exendin-4 and tetraheptylammonium bromide were each dissolved in a pH 8.0 solution and 50% methanol solution to form cations and anions. The dissolved tetraheptylammonium bromide solution was added dropwise to the exendin-4 solution to obtain hydrophobic solid compositions.

The solid compositions were added to the mixture of oil phase and hydrophilic co-solvent specified in Table 8 or the oil phase specified in Table 9 to obtain the final pharmaceutical compositions.

**[Table 8]**

| **mg/C** | | Comparative Example 7 | Comparative Example 8 | Comparative Example 9 |
|---|---|---|---|---|
| **Hydropho bic ion-pair** | exendin-4 | 1.03 | 1.03 | 1.03 |
| | Tetraheptylammonium bromide | 0.97 | 0.97 | 0.97 |
| **Total ion-pairs** | | **2.00** | **2.00** | **2.00** |
| **Oil phase** | Medium chain triglyceride | 15.0 | 60.0 | - |
| | Polyoxyl 40 hydrogenated castor oil | 40.0 | 160.0 | 160.0 |
| | Oleoyl macrogolglyceride | - | - | 60.0 |
| | Propylene glycol fatty acid ester | 41.0 | 164.0 | 164.0 |

| **Hydrophi lic co-solvent** | Propylene glycol | 4.0 | 16.0 | 16.0 |
|---|---|---|---|---|
| **Total oil phase** | | **100.0** | **400.0** | **400.0** |
| **Sum** | | **102.0** | **402.0** | **402.0** |

**[Table 9]**

| **mg/C** | | Comparative Example 10 | Comparative Example 11 |
|---|---|---|---|
| **Hydrophobi c ion-pair** | exendin-4 | 1.03 | 1.03 |
| | Tetraheptylammonium bromide | 0.97 | 0.97 |
| Total ion-pairs | | **2.00** | **2.00** |
| **Oil phase** | Medium chain triglyceride | 76.0 | - |
| | Polyoxyl 40 hydrogenated castor oil | 160.0 | 160.0 |
| | Oleoyl macrogolglyceride | - | 60.0 |
| | Propylene glycol fatty acid ester | 164.0 | 180.0 |
| **Hydrophili c co-solvent** | Propylene glycol | - | - |
| **Total oil phase** | | **400.0** | **400.0** |
| **Sum** | | **402.0** | **402.0** |

### Test Example 1: In vitro enzymatic decomposition evaluation

The pharmaceutical compositions obtained from Examples 1 to 4 and Comparative Examples 1 to 6 were tested under the conditions of Tables 10 and 11 below to evaluate whether each GLP-1 analogue was prevented from being decomposed by digestive enzymes.

**[Table 10]**

| **Details** | **Enzymatic decomposition conditions** |
|---|---|
| Enzyme solutions | USP simulated intestinal fluid |
| | USP simulated gastric fluid |
| Emulsion formation | Prepared in 5 mL purified water in an amount equivalent to 1 capsule |
| Temperature | 37°C |
| Stirring speed | 75 rpm |
| Reaction time | 5, 10, 15, 30, 45, and 60 min |
| Reagent for reaction termination | 0.1 % Trifluoroacetic acid in Ethanol |
| Centrifugation | 13,000 rpm for 10 min |

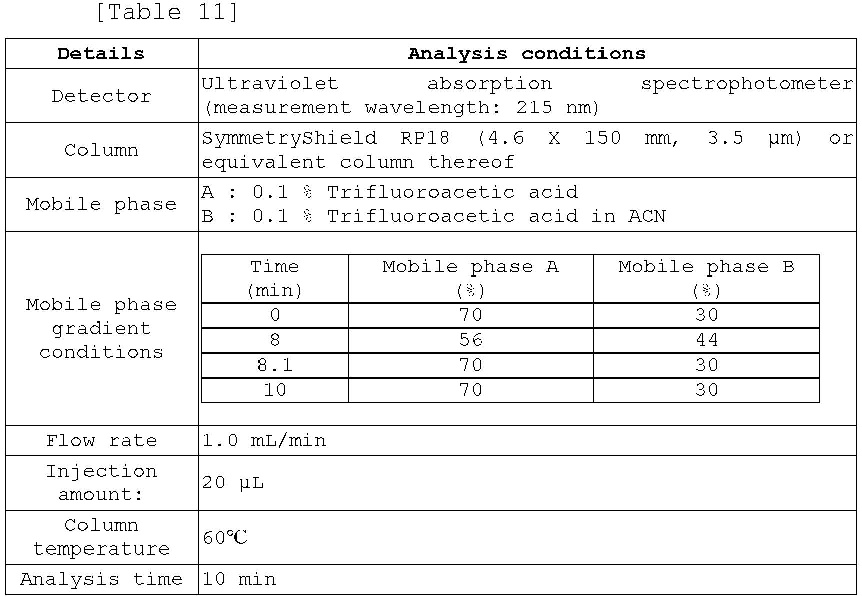

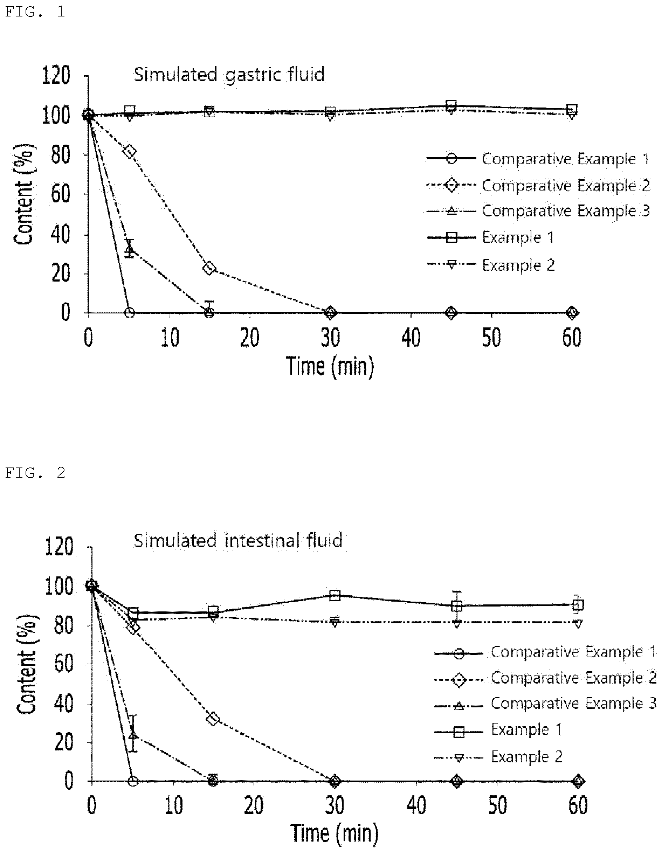

As a result of the experiment, it could be confirmed that the oil-phased compositions containing the GLP-1 analogue CA-exendin-4 bound to the hydrophobic ion-pair according to Examples 1 and 2 were stable without being decomposed by digestive enzymes (Simulated gastric fluid and simulated intestinal fluid) (FIGS. 1 and 2). These results may be obtained since the ion-pair bonding may increase the hydrophobicity of CA-exendin-4, such that CA-exendin-4 may be highly soluble in hydrophobic medium containing oil or surfactants, and thus-formed composition in which CA-exendin-4 in the form of ion-pairs is dissolved in the oil phase may form an emulsion when exposed to the digestive juice in the water phase and maintain a form thereof, blocking digestive enzymes from accessing CA-exendin-4.

On the other hand, it was confirmed in Comparative Example 1 that CA-exendin-4 was dissolved in the water phase and easily decomposed by enzymes. In addition, it could be confirmed in Comparative Example 2 that CA-exendin-4 was dispersed in a hydrophobic medium containing oil, such that even if the emulsion was formed, the CA-exendin-4 could not be encapsulated inside the emulsion, and as a result, the CA-exendin-4 was easily decomposed by simulated gastric fluid or simulated intestinal fluid. Likewise, in Comparative Example 3, it could be confirmed that the hydrophobic ion-pairs containing CA-exendin-4 were dispersed in the digestive enzyme solution to be easily decomposed by simulated gastric fluid or simulated intestinal fluid (FIGS. 1 and 2).

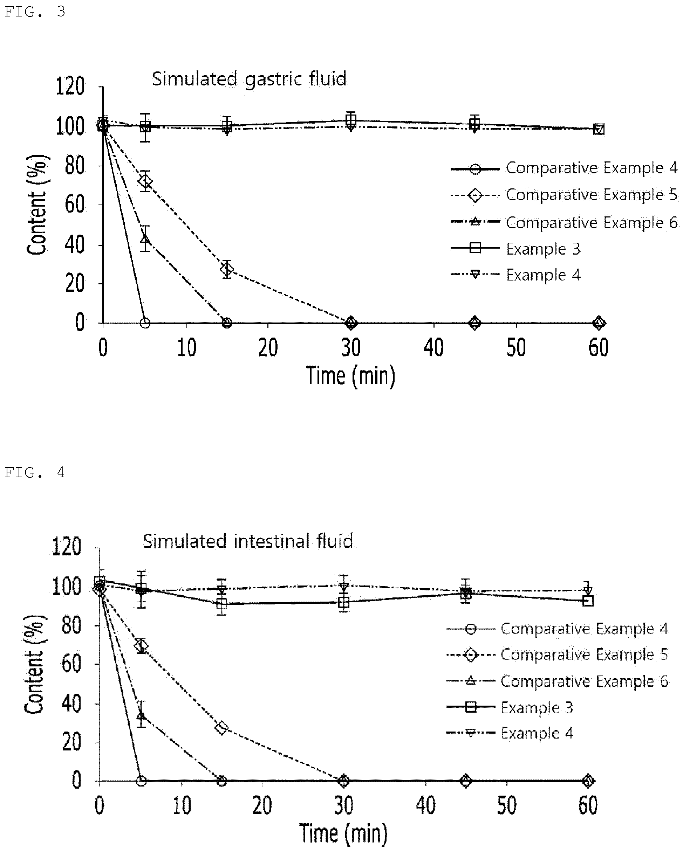

Next, as a result of experiments on exendin-4 as another GLP-1 analogue, it could be confirmed that the oil-phase compositions containing exendin-4 bound to the hydrophobic ion-pairs according to Examples 3 and 4 were also stable without being decomposed by digestive enzymes (FIGS. 3 and 4). These results are caused, similar to Examples 1 and 2, by increased hydrophobicity of exendin-4 due to ion-pair bonding, such that exendin-4 is highly soluble in hydrophobic medium containing oil or surfactants, and the emulsion form is maintained in the digestive juice in the water phase, thereby blocking digestive enzymes from accessing exendin-4.

On the other hand, it was confirmed in Comparative Example 4 that exendin-4 was dissolved in the water phase and easily decomposed by enzymes. In addition, it could be confirmed in Comparative Example 5 that exendin-4 was dispersed in a hydrophobic medium containing oil, such that even if the emulsion was formed, the exendin-4 could not be encapsulated inside the emulsion and was easily decomposed by simulated gastric fluid or simulated intestinal fluid. Likewise, in Comparative Example 6, it could be confirmed that the hydrophobic ion-pairs containing exendin-4 were dispersed in the digestive enzyme solution to be easily decomposed by simulated gastric fluid or simulated intestinal fluid (FIGS. 3 and 4).

In summary, the composition containing the GLP-1 analogue of the present disclosure may form hydrophobic ion-pairs using the GLP-1 analogue and the counter ion and contain the hydrophobic ion-pairs in the oil phase, thereby surrounding the GLP-1 analogue to form an emulsion when exposed to the water phase, unlike the GLP-1 analogue alone, the hydrophobic ion-pairs, or when included in the oil phase without formation of ion-pairs, and thus it is possible to achieve stable drug delivery without being decomposed by digestive enzymes. Therefore, the compositions of the present disclosure may be very useful for oral administration of GLP-1 analogues.

### Test Example 2: In vitro enzymatic degradation evaluation according to preparation method

Pharmaceutical compositions for oral administration obtained in Examples 5 to 7 and Comparative Examples 7 to 11 were tested under the conditions of Tables 12 and 13 below to evaluate whether GLP-1 analogues bound to ion-pairs were prevented from being decomposed by digestive enzymes.

**[Table 12]**

| **Details** | **Enzymatic decomposition conditions** |
|---|---|
| Enzyme solutions | 10.56 U/mL Pepsin in pH 1.2 (simulated gastric fluid) |
| | 2 U/mL Pancreatin in pH 6.8 (simulated intestinal fluid) |
| Sample | 2 capsules |
| Enzyme solution volume | 100 mL |
| Temperature | 37°C |
| Stirring speed | 100 rpm |
| Reaction time | 5, 10, 15, 30, 45, and 60 min |
| Reagent for reaction termination | 0.1 M NaOH for pepsin 0.1 M HCl for pancreatin |

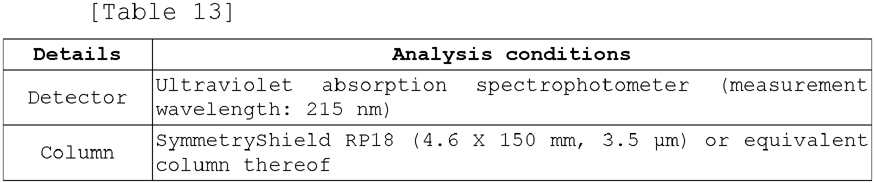

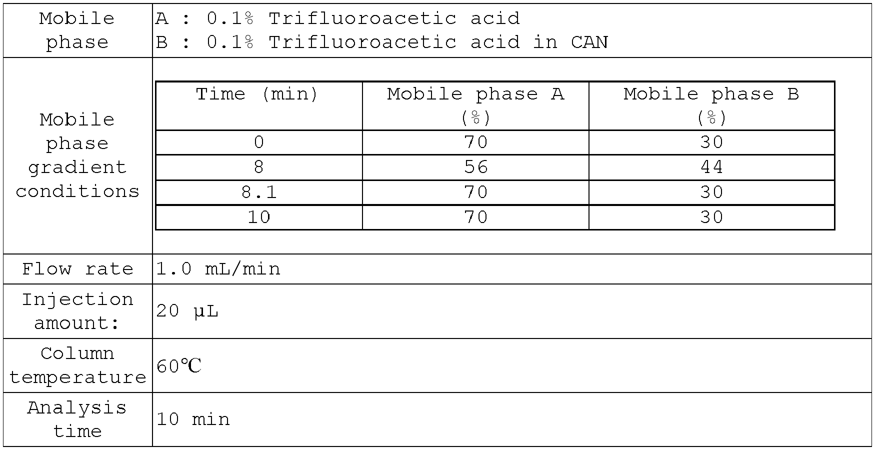

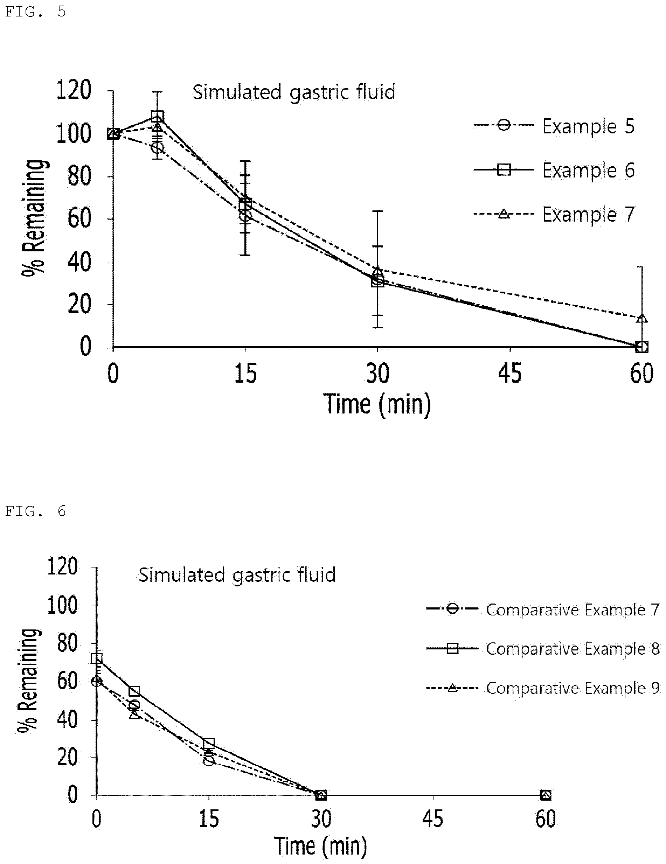

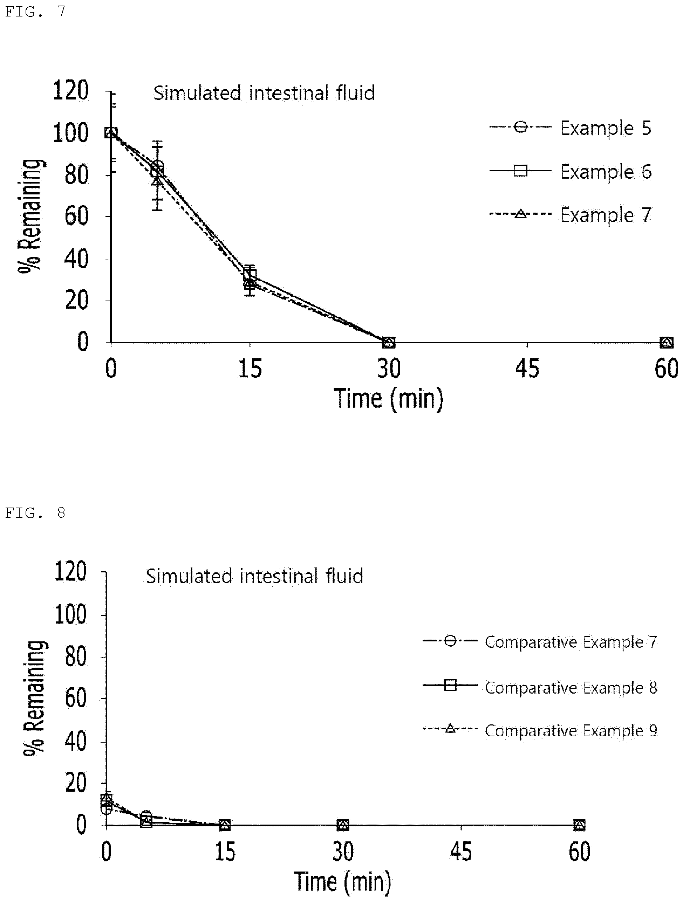

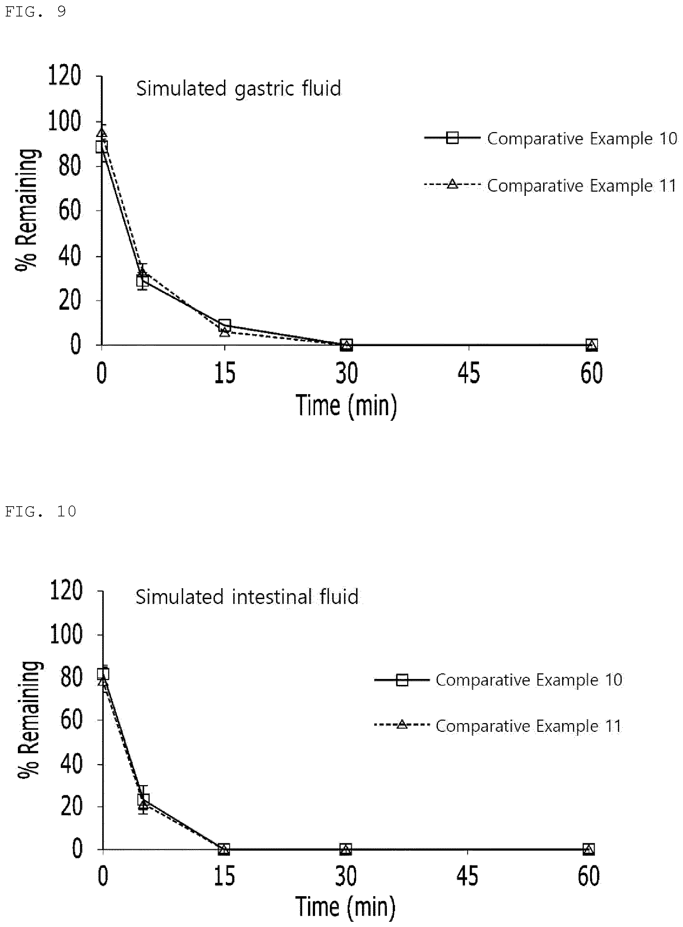

As a result, it was confirmed that Examples 5 to 7 showed stability for 30 minutes and 15 minutes or more in simulated intestinal fluid and simulated gastric fluid, respectively (FIGS. 5 and 7), but Comparative Examples 7 to 11, in which hydrophobic ion-pairs were relatively dispersed showed almost all decomposition occurred even at 0 min, and had significantly fast decomposition rates compared to Examples 5 to 7 (FIGS. 6 and 8 to 10).

This is understood to be a difference in effect that occurs depending on the degree of dispersion of the ion-pairs. When oil containing hydrophobic ion-pairs meets the water phase and forms an emulsion, the hydrophobic ion-pairs are loaded inside the emulsion and block access to enzymes. However, when the hydrophobic ion-pairs are dispersed, the compositions are exposed to digestive enzymes and quickly decomposed.

From the above description, those skilled in the art to which the present disclosure pertains will understand that the present disclosure may be embodied in other specific forms without changing the technical spirit or essential characteristics thereof. In this regard, it should be understood that the embodiments described above are illustrative in all respects and not restrictive. As the scope of the present disclosure, it should be construed that all changes or modifications derived from the meaning and scope of the claims to be described below and equivalents thereof rather than the above detailed description are included in the scope of the present disclosure.

## Claims

1. A pharmaceutical composition for oral administration comprising:
(a) a hydrophobic ion-pair formed by binding a GLP-1 analogue and a counter ion, and
(b) an oil phase containing oil or a surfactant.

2. The pharmaceutical composition for oral administration of claim 1, wherein the GLP-1 analogue is at least any one selected from the group consisting of exendin-4, CA-exendin-4 (Imidazoacetyl-exendin-4), DA-exendin-4 (Desamino-histidyl-exendin-4), HY-exendin-4 (beta-hydroxy imidazopropionyl-exendin-4), CX-exendin-4 (beta-carboxyimidazopropionyl-exendin-4), DM-exendin-4 (Dimethyl-histidyl-exendin-4), lixisenatide, liraglutide, semaglutide, dulaglutide, and albiglutide.

3. The pharmaceutical composition for oral administration of claim 1, wherein the counter ion is at least any anion selected from the group consisting of 1-hydroxy-2-naphthoic acid (xinafoic acid), 2-naphthalene sulfonic acid (NSA), brilliant blue FCF, carboxy methyl polyethylene glycol (CM-PEG), cholesteryl hemisuccinate, cholic acid, sodium cholate, decanoic acid, sodium decanoate, sodium caprate, dimyristoyl phosphatidylglycerol (DMPG), dioleoyl phosphatidic acid (DOPA), docosahexaenoic acid, hexadecyl phosphate, linoleic acid, N,N-dipalmitoyl-L-lysine, oleic acid, sodium oleate, pamoic acid, disodium pamoate, sodium acetate, sodium cholesteryl sulfate, sodium decanesulfonate (SDES), sodium deoxycholate, sodium docusate, sodium dodecyl benzenesulfonate (SDBS), sodium dodecyl sulfate, sodium laurate, sodium n-octadecyl sulfate, sodium stearate, sodium stearoyl glutamate (SSG), sodium taurodeoxycholate (STDC), sodium tetradecyl sulfate, sodium tripolyphosphate, taurocholic acid, sodium taurocholate, and vitamin E succinate.

4. The pharmaceutical composition for oral administration of claim 1, wherein the counter ion is at least any one cation selected from the group consisting of arginine-hexadecanoyl ester (AHE), arginine-nonyl ester (ANE), N-benzyl-2-phenylethanamine, chitosan, dodecylamine, hexadecyl trimethylammonium bromide (CTAB), maprotiline, N^{α}-deoxycholyl-L-lysyl-methylester, N,N'-dibenzyl ethylenediamine, N,N-dimethyl dodecylamine, N,N-dimethyl hexylamine, N,N-dimethyl octadecylamine, stearylamine, tetrabutyl ammonium bromide (TBAB), tetraheptyl ammonium bromide (THA), tetrahexyl ammonium bromide, tetraoctyl ammonium bromide (TOAB), tetrapentyl ammonium bromide (TPA), and triethylamine (TEA).

5. The pharmaceutical composition for oral administration of claim 1, wherein the oil phase containing oil or a surfactant comprises:
(a) polyoxyl castor oil having an average number of oxyethylene units of 38 or less, and
(b) at least any one selected from the group consisting of oleoyl macrogolglyceride, propylene glycol fatty acid ester, propylene glycol, diethylene glycol monoethyl ether, mono-di-glyceride, caprylocaproyl polyoxylglyceride, and polyoxyethylene sorbitan fatty acid ester.

6. The pharmaceutical composition for oral administration of claim 5, wherein the oil phase further comprises an absorption enhancer of the GLP-1 analogue.

7. The pharmaceutical composition for oral administration of claim 6, wherein the absorption enhancer is at least any one selected from the group consisting of EDTA, citric acid, salicylate, SDS, sodium deoxycholate, sodium taurocholate, oleic acid, acylcarnitine, sodium caprate, sodium caprylate, salcaprozate (SNAC), 5-CNAC, dimyristoyl phosphatidylglycerol (DMPG), cetyltrimethylammonium bromide (CTAB), and phospholipid.

8. The pharmaceutical composition for oral administration of any one of claims 1 to 7, wherein the pharmaceutical composition is for preventing or treating diabetes.

9. A method for preparing a pharmaceutical composition for oral administration, comprising:
(a) preparing a hydrophobic ion-pair by mixing a GLP-1 analogue with a counter ion; and
(b) mixing the hydrophobic ion-pair into an oil phase.

10. The method for preparing a pharmaceutical composition for oral administration of claim 9, wherein the Step (a) comprises:
(a-1) dissolving the GLP-1 analogue and the counter anion in an aqueous solution at pH 4.0, respectively;
(a-2) adding the counter anion solution dropwise to the GLP-1 analogue solution; and
(a-3) recovering the ion-pair from the mixture of the Step (a-2).

11. The method for preparing a pharmaceutical composition for oral administration of claim 9, wherein the Step (a) comprises:
(a-4) dissolving the GLP-1 analogue in an aqueous solution at pH 8.0 and dissolving the counter cation in an aqueous methanol solution;
(a-5) adding the counter cation solution dropwise to the GLP-1 analogue solution; and
(a-6) recovering the ion-pair from the mixture of the Step (a-5).

12. The method for preparing a pharmaceutical composition for oral administration of claim 9, wherein the GLP-1 analogue is at least any one selected from the group consisting of exendin-4, CA-exendin-4 (imidazoacetyl-exendin-4), DA-exendin-4 (Desamino-histidyl-exendin-4), HY-exendin-4 (beta-hydroxy imidazopropionyl-exendin-4), CX-exendin-4 (beta-carboxyimidazopropionyl-exendin-4), DM-exendin-4 (Dimethyl-histidyl-exendin-4), lixisenatide, liraglutide, semaglutide, dulaglutide, and albiglutide.

13. The method for preparing a pharmaceutical composition for oral administration of claim 9, wherein the counter ion is at least any anion selected from the group consisting of 1-hydroxy-2-naphthoic acid (xinafoic acid), 2-naphthalene sulfonic acid (NSA), brilliant blue FCF, carboxy methyl polyethylene glycol (CM-PEG), cholesteryl hemisuccinate, cholic acid, sodium cholate, decanoic acid, sodium decanoate, sodium caprate, dimyristoyl phosphatidylglycerol (DMPG), dioleoyl phosphatidic acid (DOPA), docosahexaenoic acid, hexadecyl phosphate, linoleic acid, N,N-dipalmitoyl-L-lysine, oleic acid, sodium oleate, pamoic acid, disodium pamoate, sodium acetate, sodium cholesteryl sulfate, sodium decanesulfonate (SDES), sodium deoxycholate, sodium docusate, sodium dodecyl benzenesulfonate (SDBS), sodium dodecyl sulfate, sodium laurate, sodium n-octadecyl sulfate, sodium stearate, sodium stearoyl glutamate (SSG), sodium taurodeoxycholate (STDC), sodium tetradecyl sulfate, sodium tripolyphosphate, taurocholic acid, sodium taurocholate, and vitamin E succinate.

14. The method for preparing a pharmaceutical composition for oral administration of claim 9, wherein the counter ion is at least any one cation selected from the group consisting of arginine-hexadecanoyl ester (AHE), arginine-nonyl ester (ANE), N-benzyl-2-phenylethanamine, chitosan, dodecylamine, hexadecyl trimethylammonium bromide (CTAB), maprotiline, N^{α}-deoxycholyl-L-lysyl-methylester, N,N'-dibenzyl ethylenediamine, N,N-dimethyl dodecylamine, N,N-dimethyl hexylamine, N,N-dimethyl octadecylamine, stearylamine, tetrabutyl ammonium bromide (TBAB), tetraheptyl ammonium bromide (THA), tetrahexyl ammonium bromide, tetraoctyl ammonium bromide (TOAB), tetrapentyl ammonium bromide (TPA), and triethylamine (TEA).

15. The method for preparing a pharmaceutical composition for oral administration of claim 9, wherein the oil phase containing oil or a surfactant comprises:
(a) polyoxyl castor oil having an average number of oxyethylene units of 38 or less, and
(b) at least any one selected from the group consisting of oleoyl macrogolglyceride, propylene glycol fatty acid ester, propylene glycol, diethylene glycol monoethyl ether, mono-di-glyceride, caprylocaproyl polyoxylglyceride, and polyoxyethylene sorbitan fatty acid ester.

16. The method for preparing a pharmaceutical composition for oral administration of claim 15, wherein the oil phase further comprises an absorption enhancer of the GLP-1 analogue.

17. The method for preparing a pharmaceutical composition for oral administration of claim 16, wherein the absorption enhancer is at least any one selected from the group consisting of EDTA, citric acid, salicylate, SDS, sodium deoxycholate, sodium taurocholate, oleic acid, acylcarnitine, sodium caprate, sodium caprylate, salcaprozate (SNAC), 5-CNAC, dimyristoyl phosphatidylglycerol (DMPG), cetyltrimethylammonium bromide (CTAB), and phospholipid.

18. A pharmaceutical composition for oral administration prepared by the method of any one of claims 9 to 17.

19. A method for preparing a pharmaceutical composition for oral administration, comprising:
(a) preparing a hydrophobic ion-pair by mixing a GLP-1 analogue with a counter ion;
(b) adding the hydrophobic ion-pair to a mixture of medium chain triglyceride and propylene glycol; and
(c) adding and mixing at least any one selected from the group consisting of polyoxyl 35 castor oil, polyoxyl 40 hydrogenated castor oil, oleoyl macrogolglyceride, and propylene glycol fatty acid ester, to a mixture prepared in the Step (b).

20. A method for preparing a pharmaceutical composition for oral administration, comprising:
(a) preparing a hydrophobic ion-pair by mixing a GLP-1 analogue with a counter ion;
(b) adding the hydrophobic ion-pair to a mixture of propylene glycol fatty acid ester and propylene glycol; and
(c) adding and mixing at least any one selected from the group consisting of medium chain triglyceride, polyoxyl 35 castor oil, polyoxyl 40 hydrogenated castor oil, and oleoyl macrogolglyceride, to a mixture prepared in the Step (b) .

21. A pharmaceutical composition for oral administration prepared by the method of claim 19 or 20.
